# EUROPEAN PATENT SPECIFICATION

(11) **EP 2 285 281 B1**
(45) Date of publication and mention of the grant of the patent: **28.09.2011**
(21) Application number: 09757920.5
(22) Date of filing: 26.05.2009
(51) Int. Cl.: A61B 6/00

(54) **DEVICE FOR A COMPUTER TOMOGRAPHY GANTRY FOR TRANSMITTING DATA**
VORRICHTUNG FÜR EIN COMPUTERTOMOGRAPHIEGERÜST ZUR DATENÜBERTRAGUNG
DISPOSITIF POUR UN PORTIQUE DE TOMOGRAPHIE INFORMATIQUE POUR LA TRANSMISSION DE DONNÉES

(30) Priority: 02.06.2008 EP 08104199
(43) Date of publication of application: 23.02.2011
(73) Proprietor: Philips Intellectual Property & Standards GmbH, 20099 Hamburg (DE); Koninklijke Philips Electronics N.V., 5621 BA Eindhoven (NL)
(72) Inventor: URBAN, Max, C., NL-5656 AE Eindhoven (NL); SEIFARTH, Christoph, NL-5656 AE Eindhoven (NL); SCHOLL, Gerd, J., NL-5656 AE Eindhoven (NL); VAN LIEROP, Hendrikus, W., L., A., M., NL-5656 AE Eindhoven (NL)
(74) Representative: Van Velzen, Maaike Mathilde
(86) International application number: PCT/IB2009/052189
(87) International publication number: WO 2009/147569

(56) References cited:
- WO-A-2007/034357
- DE-A1-102005 022 825
- US-A- 4 796 183
- US-A- 5 530 425
- US-A1- 2001 016 032
- US-A1- 2007 086 782

## Description

### FIELD OF THE INVENTION

The present invention relates to a device for a computer tomography gantry for transmitting data and a computer tomography gantry comprising such a device.

### BACKGROUND OF THE INVENTION

In X-ray image acquisition using a computer tomography device with a rotatable gantry, a precise measurement of a gantry position and speed is very important for a correct image reconstruction. In former systems, the rotation position and speed is measured by counting marks on the gantry rotor. This method may be of low quality with respect to the precision. As a result thereof the image processing of the computer tomography system may suffer because of deficits in detecting a precise position of a rotary part of the gantry as well as a precise rotation speed of the rotary part of the gantry with respect to a stationary part of the gantry.

US-A-4 796 183 discloses a data transmission device for a computer tomography gantry. The device includes a waveguide to which microwave transmitters are attached. The microwave transmitters transmit electromagnetic radiation into the waveguide in a clockwise and counterclockwise direction. Symmetrically disposed wavesinks divide the waveguide into sections of identical length. The waveguide, which constitutes a data transmission path, is thus divided into sub-paths by the wavesinks. Data transmission proceeds from the transmitters to receivers which are offset relative to each other. As a result of selected different numbers of the transmitters and receivers continuous transmission of data is assured because one transmitter and one receiver always communicate with each other via a data link consisting of adjoining sub paths.

### SUMMARY OF THE INVENTION

It may be desirable to provide an improved device for detecting the position and rotation speed of the rotary part of the gantry as well as an improved arrangement to transmit data between the rotary part of the gantry and the stationary part of the gantry in order to enable a wide-band transmission.

According to a first aspect, the invention provides a device for a computer tomography gantry for transmitting data, wherein the gantry comprises a stationary part and a rotary part,
wherein the device is adapted to transmit the data between the stationary part of the gantry and the rotary part of the gantry. Therein, the device comprises a hollow conductor which is adapted to guide a first wave, a sender which is adapted to send the first wave inside the hollow conductor, and a receiver which is adapted to receive the first wave after a runtime inside the hollow conductor. The device is further adapted to transmit a second wave inside the hollow conductor, wherein the first wave runs clockwise and the second wave runs counter-clockwise, and the device is adapted to measure the runtimes of the first wave and the second wave.

According to a second aspect, the invention provides a computer tomography (CT) gantry comprising a device according to the above first aspect.

It may be seen as a gist of the present invention to use a hollow conductor for precise measurement of the position and/or the rotation speed of a rotary part e.g. of a gantry of a CT device. The hollow conductor can be provided either on the stationary part or on the rotary part of the gantry. A sender may emit an electromagnetic wave into the hollow conductor. The electromagnetic wave may travel along the hollow conductor and may finally be received by a receiver. Therein, the sender may be provided on one of the stationary and the rotary part of the gantry whereas the receiver may be provided on the other of the stationary and the rotary part of the gantry. There may be one or more senders and one or more receivers. A sender and a receiver may be combined in a transceiver. Measuring characteristics of the transmitted wave such as for example a time-of-flight from the sender to the receiver, interferences, beat-detection or a Doppler-shift may enable to precisely detect a current position of the receiver relative to the sender thereby allowing to precisely determine a relative position and rotation speed of the rotary part of the gantry with respect to the stationary part of the gantry. This may result in improving an image processing of the computer tomography system.

Furthermore, as another essence of the invention, a broadband transmission of data may be provided with the help of the hollow conductor. Therein, for example control data may be provided from a control located at the stationary part to for example an X-ray tube located at the rotary part by a data transmission from the sender to the receiver. Therein, the data may be transmitted contactless, i.e. without any mechanical contact between the sender and the receiver.

Typically, the hollow conductor, also referred to as waveguide, may be realized with lateral surfaces which incorporate a volume. Usually, this volume is air-filled. The included electromagnetic waves will be reflected by the lateral surfaces in order to keep them in the hollow conductor. One of the lateral surfaces may be slotted along its length. This slot may render the possibility for extending a sender, a receiver and/or an antenna into the hollow conductor.

Further embodiments are incorporated in the dependent claims.

According to the present invention it is provided a device, wherein the hollow conductor is arranged at one of the stationary part and the rotary part of the gantry, wherein the gantry comprises an inner bore, wherein the hollow conductor is arranged around the inner bore of the gantry.

In this situation a sender or a receiver is arranged at the rotary part of the gantry and the hollow conductor is arranged at the stationary part of the gantry. The hollow conductor may be a tubular ring having a slit along its circumference and being arranged concentrically with the gantry. The hollow conductor comprises at least one further sender, receiver, antenna, e.g. extending from the rotary part. The opposite arrangement is also possible, i.e. that the hollow conductor is arranged at the rotary part of the gantry.

According to the invention it is provided a device wherein the device is adapted to transmit a second wave inside the hollow conductor, wherein the first wave runs clockwise and the second wave runs counterclockwise, wherein the device is adapted to measure the runtimes of the first wave and the second wave.

Herein, the runtimes refer to the duration the first and the second wave need to come from the sender to the receiver. These runtimes and, in particular, differences between the runtime of the first wave and the runtime of the second wave may be used to determine the position and/or the rotational speed of the rotary part with respect to the stationary part of the gantry.

According to the present invention it is provided a device wherein the device is adapted to calculate or determine the distance between the receiver and the sender by processing the runtimes of the first wave and the second wave.

Therein, the distance between the receiver and the sender may refer to a distance along the travel path of the first and second waves. In other words, as the waves travel along the hollow conductor which itself is mounted to one of the stationary and the rotary part of the gantry, the determined distance between the receiver and the sender may indicate a relative position between the rotary part and the stationary part. This relative position may also indicate an angular position of the rotary part with respect to the stationary part.

According to an exemplary embodiment it is provided an X-ray device, wherein the device is adapted to repeat the calculation or determination of the distance, wherein the device is adapted to calculate the rotation speed of the rotary part of the gantry.

In other words, the distance between the sender and the receiver is repeatedly determined at different points in time and from the changes of the determined distances and the time between the respective determinations the rotation speed of the rotary part of the gantry may be determined.

The measurement according to this concept may lead to precise results of the position and the rotation speed of the rotary part of the gantry. These precise results may have a positive impact on the whole image processing of the computer tomography system.

According to an exemplary embodiment it is provided an X-ray device, wherein the first wave comprises data, wherein the device is adapted to receive the first wave after a runtime inside the hollow conductor, wherein the device is adapted to extract the data from the first wave after a runtime of the first wave in the hollow conductor.

The data transmission with the help of the hollow conductor renders the possibility for a broadband transmission of data. Therein, data may be transmitted from the stationary part to the rotary part of the gantry or vice versa without the need for mechanical or electrical connection between the rotary part and the stationary part. Independent of the current rotational position and speed of the rotary part, data can be transmitted with a high data transmission rate of for example 8 bits/2 µs from the sender arranged at one of the stationary and the rotary part to the receiver arranged at the other of the stationary and the rotary part of the gantry. Thereby, additional to a wireless power transfer between the stationary and the rotary part of the gantry, an extra data link can be provided to control for example the voltage supplied to an X-ray tube arranged on the rotary part of the gantry.

According to an exemplary embodiment it is provided an X-ray device, wherein the hollow conductor is adapted to dampen the first wave, wherein the hollow conductor is adapted to diminish the amplitude of the first wave after one circulation or revolution around the hollow conductor considerably.

The damping of the first wave travelling within the hollow conductor should be such that undesired reflections or interferences of waves running around the circular hollow conductor several times are essentially suppressed. For example, the amplitude of a wave having travelled once around the hollow conductor should be clearly distinguishable from the amplitude of a wave having travelled twice around the hollow conductor.

According to another exemplary embodiment it is provided an X-ray device, wherein the hollow conductor comprises a material, which is adapted to diminish the amplitude of the first wave after one circulation around the hollow conductor considerably.

In other words, the characteristics of a material forming the hollow conductor may be such that a wave travelling within the hollow conductor is considerably dampened thereby diminishing its amplitude. For example, the material of the hollow conductor may have a sufficiently low electrical conductivity.

According to another exemplary embodiment it is provided an X-ray device, wherein the hollow conductor is at least partly coated in the interior zone.

For example, the hollow conductor may comprise a highly electrically conducting base material which is coated with a material having low electrical conductivity and/or having a high damping factor. Alternatively, the base material of the hollow conductor may be electrically non-conducting and may be coated with a material of low electrical conductivity.

According to an exemplary embodiment it is provided an X-ray device, wherein the coating of the hollow conductor is homogeneous. This means that at least a part of the coating may be of similar, especially uniform, chemical composition.

The damping can be achieved by using such a material for the construction of the hollow conductor, which is characterised by damping waves. Another possibilty is the coating of a part of the interior zone of the hollow conductor. It is also possible to coat the whole area of the interior zone. Usually the coating is homogeneous. But it is also possible that the coating differs along the hollow conductor.

According to an exemplary embodiment it is provided an X-ray device, wherein the hollow conductor is adapted to diminish the amplitude of the first wave with a first frequency after one circulation around the hollow conductor considerably, wherein the hollow conductor is adapted not to diminish the amplitude of a second wave with a second frequency after one circulation around the hollow conductor considerably, wherein the first frequency is different to the second frequency.

According to another exemplary embodiment it is provided an X-ray device, wherein a side surface of the hollow conductor is closed.

According to an exemplary embodiment it is provided an X-ray device, wherein the hollow conductor is ring-shaped.

According to another exemplary embodiment it is provided an X-ray device, wherein the hollow conductor is a slotted hollow conductor also referred to as slotted waveguide.

According to another exemplary embodiment, the device is adapted to determine a relative position between the stationary part and the rotary part based on a measurement of the first wave running from the sender to the receiver.

For example, the receiver is arranged at the stationary part of the gantry whereas the sender is arranged on the rotary part of the gantry. A rotation angle in between the receiver and the sender may be described as time-dependent angle α(t). A variation in this angle α(t) may introduce a small variation in a delay of data transmitted between the sender and the receiver. As the electromagnetic wave travels in the hollow conductor at the speed of light, i.e. at approximately 3 x 10⁸ m/s, and because the circumference of a typical gantry is for example 3.8 m, a maximum delay may be approximately 6.3 ns when the sender and the receiver are 180° apart. Measuring this delay may reveal a rotational angle between the sender and the receiver. This may be interpreted as the well-known time-of-flight principle. State-of-the-art electronics should be able to measure the delay with picosecond resolution, resulting in an angular resolution of typically 0.03° (1 ps/6.3 ns x 180°). This may be about three times better than the resolution of a resolver that is conventionally being employed for measuring rotational angles. Instead of using a plain time-of-flight principle, one could also use interference, beat-detection or the Doppler-shift to measure the angular position or speed of the parts of the gantry with respect to each other.

According to another exemplary embodiment, the sender and the receiver are both provided at the same one of the stationary part and the rotary part. The sender and the receiver may be provided as one single component referred to as transceiver. A reflector is provided at the other of the stationary part and the rotary part. Then, the device may be adapted to determine a relative position between the stationary part and the rotary part based on a measurement of the first wave running from the sender to the reflector and back to the receiver.

In this specific embodiment, it may not be necessary to actually transmit data from the sender to the receiver as both, sender and receiver, are arranged at the same part of the gantry. Instead, a simple signal can be emitted by the sender in a direction towards the reflector. At the reflector, this signal is reflected and travels back to the receiver. Using such arrangement with a reflector, the distance of the travel path of the wave is doubled thereby reducing the requirements for the time resolution of a detection circuit. In other words, doubling the distance travelled by the electromagnetic waves may result in halving the required resolution.

According to another exemplary embodiment, the device comprises two receivers and an electromagnetic shield is positioned between the two receivers. A first receiver is adapted to detect a first wave running from the sender to the first receiver in a clockwise direction and a second receiver is adapted to detect a second wave running from the sender to the second receiver in a counter-clockwise direction.

In other words, the bi-directional propagation of waves through a circular hollow conductor or waveguide, i.e. propagation in both the clockwise (CW) and the counter-clockwise (CCW) direction, may be used to determine the relative position of the sender and the receiver. While it may be difficult for a single receiver to distinguish between receiving the clockwise travelling wave and receiving the counter-clockwise travelling wave, it may be advantageous to use two receivers in between which there is an electromagnetic shield. The electromagnetic shield may be adapted to block or at least to significantly dampen the transmission of an impacting electromagnetic wave. In this way, there may be a dedicated receiver for each electromagnetic wave, clockwise and counter-clockwise, making it easier to discern the waves for an electronic circuitry.

It should be noted that the above features may also be combined. The combination of the above features may also lead to synergetic effects, even if not explicitly described in detail.

These and other aspects of the present invention will become apparent from and elucidated with reference to the embodiments described hereinafter.

### BRIEF DESCRIPTION OF THE DRAWINGS

Exemplary embodiments of the present invention will be described in the following with reference to the following drawings.
Fig. 1 shows a side view onto a hollow conductor for an X-ray device and a diagram of intensities,
Fig. 2 shows a side view onto a hollow conductor for an X-ray device,
Fig. 3 shows a cross section of a hollow conductor arranged at a transformer for an X-ray device,
Fig. 4 shows a side view onto a specific hollow conductor for an X-ray device,
Fig. 5 shows a side view on another specific hollow conductor for an X-ray device;
Fig. 6 shows a circuitry for measuring an angular position in a computer tomography gantry,
Fig. 7 shows a perspective view of a computer tomography gantry.

The drawings in the figures are only schematically and not to scale. Similar components are indicated with similar reference signs throughout the figures.

### DETAILED DESCRIPTION OF EMBODIMENTS

In a rotation system, e.g. a CT system gantry, round, closed and slotted hollow conductors (waveguides) can be used for low latency or response-time free data transfer between rotating and non rotating side. A pulse-patterned wave with a defined frequency can be sent by the sender. A receiver measures this signal, either as two single pulses or as one pulse with a very high amplitude. Depending on the position of the sender of the gantry these alternating kinds of pulses can occur. A high precision rotation speed measurement is possible either by intensity measurement of the received pulses or by a detuning of sender and receiver frequency and measuring a interference pulse.

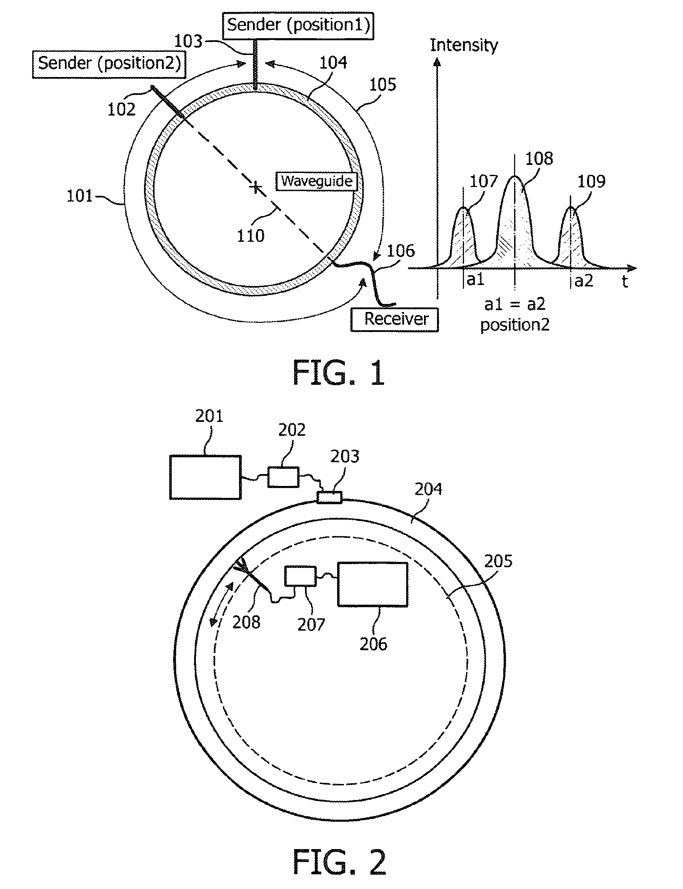

Fig. 1 shows a hollow conductor (waveguide) 104, wherein two different positions of the same sender (position 1, position 2) 102, 103 are depicted. Starting from the sender with the position 1 103 a first wave runs clockwise to the receiver 106 and a second wave runs counterclockwise to the receiver 106. With the processing of the runtimes of the first wave and the second wave, which are detected by the receiver 106 it is possible to measure the position 1 103 of the sender. The diagram of the intensities shows a situation 108 in which the sender is opposed to the receiver 106 (180 degrees, position 2, 102). The dotted line 110 shows the situation that the sender 102 is arranged directly opposed to the receiver 106. This situation results in the intensity 108, detected by the receiver 106. The dotted line 110 passes the center of the hollow conductor 104. The intensities 107 and 109 show the situation which is detected by the receiver 106 when the sender is in a position 1 103.

In Fig. 1 a waveguide ring 104 with one sender 102, 103 and one receiver 106 is shown. A signal, which is coded in a microwave and which is sent, runs along both sides of the waveguide ring. Two pulses 107, 109 can be measured by the receiver. If sender and receiver are at opposite positions (180°), both pulses have the same runtime and only one resulting pulse 108 (addition of both pulses) is measured. The time between one pulse and the maximum distance of both pulses gives an indication of the rotation speed. More easily the time between two "one-pulse-is-measured" situations can be measured. That's the time for one rotation.

Advantageously, the rotation speed can be measured without additional mechanical components (like slits or counting marks). According to the inventive concept a higher precision measurement of the position and the rotation speed of the gantry is possible than with former systems by the use of signal-run time or interference patterns.

Fig. 2 shows a hollow conductor 204, and the rotary part of a gantry 205. It is also depicted a data sender/receiver 203 with processing units 202 and 201. It is also depicted a sender/receiver 208 with means for processing of data 207, 206.

The Fig. 2 shows the rotary part of a gantry 205 with a hollow conductor 204 in which a wave can be guided. This renders the possibility to transmit data inside the hollow conductor between the rotary part of the gantry to the stationary part of the gantry without a mechanical connection between the rotary part of the gantry and the stationary part of the gantry. It is possible to arrange a sender or a receiver at a stationary part of the gantry and to arrange a sender or receiver at the rotary part of the gantry. Therefore, data can be transmitted in both directions, data can be transmitted from the stationary part to the rotary part of the gantry and data can be transmitted from the rotary part to the stationary part of the gantry. It is especially advantageous to arrange periodically i.e. equidistant, or not periodically dampings or an absorption structure in the interior zone of the hollow conductor 204. It is also possible to arrange deficits in a dielectricum or slots in the hollow conductor in order to dampen the wave, circulating in the hollow conductor.

A unit 206 supplies an antenna 208 with data, wherein the sender transmits a wave in the hollow conductor 204. The hollow conductor dampens the wave in such a way, that the wave is considerably dampened after a first circulation in the hollow conductor 204. This leads to the effect, that because of the reduced amplitude of the wave after a circulation it is possible to filter waves after one or several circulations in the hollow conductor 204. The receiver 203 detects the wave. Further, the data which is encoded in the wave can be decoded by processing units 202, 201. Therefore, this device offers the possibility to transmit data between the rotary part of a gantry at a stationary part of the gantry without a mechanical connection between the rotary part of the gantry and the stationary part of the gantry. This device renders also the possibility to transmit data without interruption between the rotary part of the gantry and the stationary part of the gantry.

The hollow conductor 204 is typically ring-shaped and at least one lateral surface of the hollow conductor 204 is closed. Advantageously the hollow conductor 204 is slotted and filled with air. The hollow conductor 204 comprises at least one sender and one receiver, wherein it is also possible that the hollow conductor 204 comprises two units, which comprise both functions of a sender and a receiver. Due to this fact, data can be transmitted from the stationary part to the rotary part of the gantry and vice versa. It is also possible to arrange several senders or several receivers in the hollow conductor 204, wherein the units can fulfil both functions of a sender and a receiver. Advantageously the hollow conductor dampens the waves which were sent by the sender, in order to dampen the waves after a first circulation. This can be realized for example by materials of the hollow conductor, which conduct badly. A further example to arrive at a dampening hollow conductor 204 is the use of coating the interior zone of the hollow conductor 204.

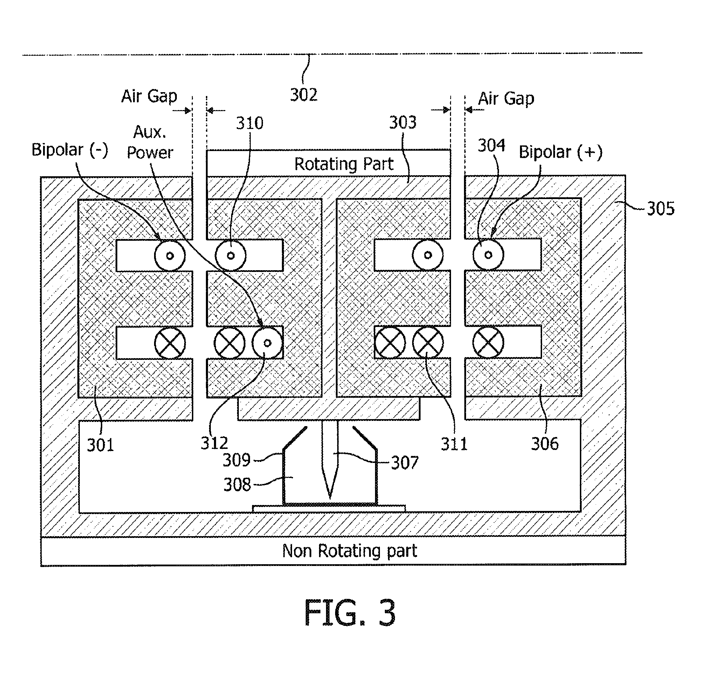

Fig. 3 shows a cross-section of a transformer which may be used, on the one hand, to supply power from a stationary part 305 to a rotary part 303 of a gantry, and on which, on the other hand, provisions are made for data submission from the stationary part to the rotary part via a slotted hollow conductor 308. It is depicted the primary side of the transformer 305 with a E-shaped core and the secondary side of the transformer 303, which is the rotating part of the transformer. It is also depicted windings 304, 311, 312, 310 of the transformer. The secondary part of the transformer (rotating part) 303 is rotating around the center line 302. It is arranged a unit for sending or receiving or of both functions of sending and receiving 307 at the secondary side of the transformer 303. With the help of the unit 307 it is possible to transmit data inside the hollow conductor 308. The unit 307 enables also the possibility to detect the position of the secondary side of the transformer as well as the speed of the rotation of the secondary side of the transformer 303. The hollow conductor 308 is filled with air. The hollow conductor 308 is slotted in order to enable the unit 307 to extend into the hollow conductor 308. The hollow conductor 308 comprises five lateral surfaces 309. two of the five lateral surfaces 309 are bent in order to enable the sender/receiver/antenna 307 to extend into the hollow conductor 308.

The device according to the inventive concept is adapted to be able to be used without a mechanical connection between the rotary part of the gantry and the stationary part of the gantry. Due to that fact the hollow conductor (waveguide) can be regarded as maintenance-free.

When building a waveguide link system, several data slots (at different frequencies) are available. One frequency (data slot) of the slotted waveguide has to be used for the rotation-speed-pulse-pattern. The receiver is able to detect this pattern and can measure the runtime. To analyze/evaluate/interpret the rotation-speed-pulse-pattern various methods are possible. Either a Fourrier transformation of the pulse-run-time can be implemented in a processor and gives a frequency spectrum which contains a peak. The height depends on the rotation speed (constructive interference). The time between two maxima is the time of one gantry revolution. Another possible evaluation is the analysis of the runtime of both signals. A Kalman filter model can be used to predict the runtime. If the predicted runtime matches with the measured runtime, a given gantry speed is achieved.

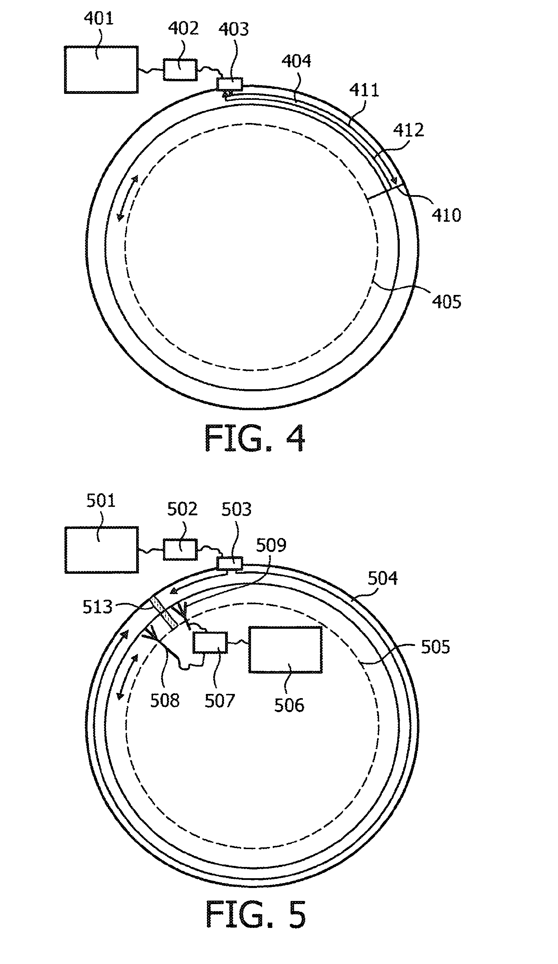

Fig. 4 shows a specific embodiment of a rotary part of a gantry 405 with a hollow conductor 404 arranged thereon. A transceiver 403 serving as both a receiver and a sender is provided at a stationary part of the gantry and is connected to processing units 401, 402. The hollow conductor 404 is provided with a reflector 410 which is adapted for reflecting electromagnetic waves travelling within the hollow conductor. The reflector 410 rotates together with the rotating part of the gantry 405.

Electromagnetic waves emitted by the transceiver 403 travel in a clockwise direction indicated by the arrow 411 towards the reflector 410. There, the waves are reflected and travel back in a counter-clockwise direction, indicated by the arrow 412, towards the transceiver 403 where they are detected. Using a time-of-flight principle involves such reflection of the electromagnetic waves to enable collocation of the sender and the receiver in a transceiver 403. This doubles the distance travelled by the electromagnetic wave, halving the required resolution of the processing unit 401, 402 and allowing using the same timing signal for both the sender and the receiver. Thereby, the need for synchronization of two separate clocks may be eliminated.

A circular waveguide or hollow conductor 404 will usually guide the electromagnetic wave emitted by the transceiver 403 in two directions, i.e. both clockwise and counter-clockwise. Although not specifically indicated in Fig. 4, the electromagnetic wave emitted by the transceiver 403 may also first travel in a counter-clockwise direction before being reflected at the reflector 410 and travelling back in a clockwise direction towards the transceiver 403.

Depending on the relative positions of the transceiver 403 and the reflector 410, one wave will arrive earlier than the other. The difference in time of arrival is the only measurement that is needed. In the embodiment shown in Fig. 4, only the transceiver 403 needs an accurate clock. In the embodiment shown in Fig. 2, only the receiver 208 needs such accurate clock. This clock does not need to provide an absolute time nor does it need to be synchronized but only needs to measure a difference in time between the arrival of a wave originally emitted in a clockwise direction and the time of arrival of a wave originally emitted in a counter-clockwise direction.

Fig. 5 shows another specific embodiment of a rotary part of the gantry 505 provided with a hollow conductor 504. Two separate receivers 508, 509 are provided on the rotary part and connected to processing units 506, 507. An electromagnetic shield 513 is provided between the receivers 508, 509. The electromagnetic shield 513 is adapted and arranged in the hollow conductor 504 such that electromagnetic waves coming from a sender 503 cannot be transmitted through the shield 513. Accordingly, as shown in Fig. 5, an electromagnetic wave travelling in a counter-clockwise direction is received by the receiver 509 whereas an electromagnetic wave travelling in the clockwise direction is received with the receiver 508. This makes it easier to discern the wave with the circuitry 506, 507.

When simply sending a pulse or a train of pulses, the required resolution is in the picosecond range. If however continuous sinusoidal waves are being used, one can apply beat detection, phase-shift measurement or may be even Doppler-shift measurement. These commonly used methods may relax the constraints on the timing circuitry.

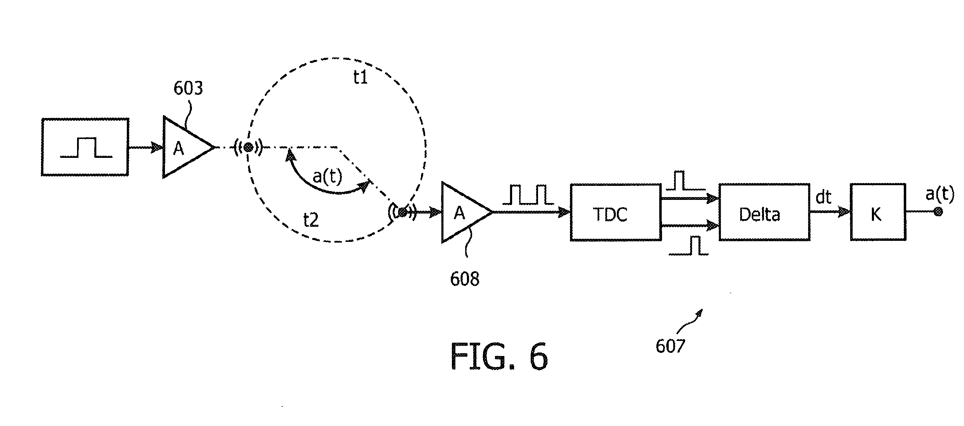

As schematically indicated by the circuitry shown in Fig. 6, when using a plain pulse, an accurate timing discriminator (TDC) is required. For example, a rectangular pulse may be emitted by a sender 603 on a stationary part of a gantry. The wave signal may be detected by a receiver 608 arranged at the rotary part of the gantry. A timing discriminator being part of a processing unit 607 may accurately detect the rising slope of a pulse applied to its input port. Two pulses corresponding to a wave transmission in a clockwise direction and a wave transmission in a counter-clockwise direction may be detected shortly after each other and the difference in their time-of-arrival may be used to calculate the angular position α(t).

In an alternative embodiment, a carrier signal that is being used to establish the data link between the stationary and the rotary part of a gantry is also used to calculate the difference in the time-of-arrival. An interference of the two opposite waves (CW), (CCW) contains information on the angular position of the sender/receiver. This information can be extracted accurately through interpolation and using well-known interferometric principles. Ideally, in such case, (a) the electromagnetic wave is absorbed at the antenna for example by an additional absorber inserted between two receiving antennas to prevent the wave from making many revolutions which could otherwise disturb the measurement, or (b) the circumference measures a multiple of the wavelength of the carrier wave, creating a standing wave.

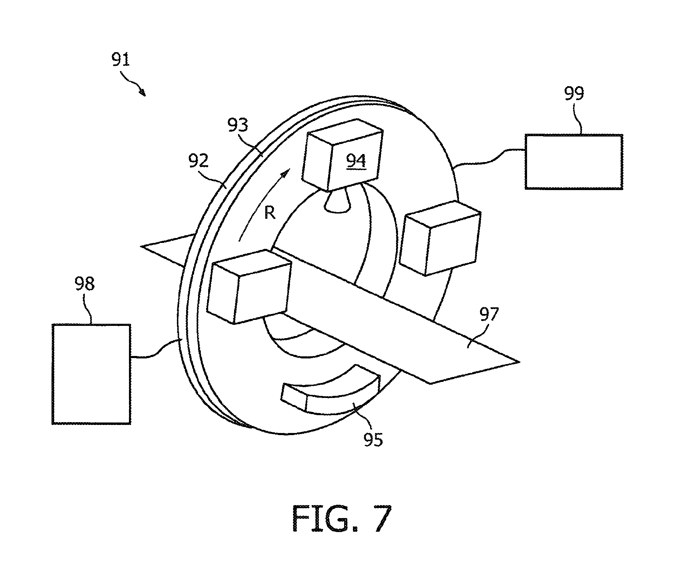

Fig. 7 shows an exemplary embodiment of a computer tomography gantry 91 arrangement. The gantry 91 comprises a stationary part 92 connected to a high frequency power source 98 and a rotary part 93 adapted to rotate relative to the stationary part 92. An X-ray source 94 and an X-ray detector 95 are attached to the rotary part 93 at opposing locations such as to be rotatable around a patient positioned on a table 97. The X-ray detector 95 and the X-ray source 94 are connected to a control and analysing unit 99 adapted to control the X-ray detector 95 and the X-ray source and to evaluate the detection results of the X-ray detector 95.

It has to be mentioned that the wording sender can be replaced by the wording transmitter.

It should be noted that the term 'comprising' does not exclude other elements or steps and the 'a' or 'an' does not exclude a plurality. Also elements described in association with the different embodiments may be combined.

It should be noted that the reference signs in the claims shall not be construed as limiting the scope of the claims.

### LIST OF REFERENCE SIGNS

- 91: Computer tomography gantry
- 92: Stationary part of the gantry
- 93: Rotary part of the gantry
- 94: X-ray source
- 95: X-ray detector
- 97: Table
- 98: High frequency power source
- 99: Control and analysing unit
- 101: Distance
- 102: Sender
- 103: Sender
- 104: Hollow conductor
- 105: Distance
- 106: Receiver
- 107: Intensity
- 108: Intensity
- 109: Intensity
- 201: Processing unit
- 202: Processing unit
- 203: Receiver/sender
- 204: Hollow conductor
- 205: Rotary part of a transformer
- 206: Processing unit
- 207: Processing unit
- 208: Receiver/sender
- 301: Core
- 302: center line
- 303: Rotary part of a transformer
- 304: Winding
- 305: Stationary part of a transformer
- 306: Core
- 307: Sender/Receiver
- 308: Hollow conductor
- 309: Lateral surface of a hollow conductor
- 310: Winding
- 311: Winding
- 312: Winding
- 401: Processing unit
- 402: Processing unit
- 403: Transceiver
- 404: Hollow conductor
- 405: Gantry
- 410: Reflector
- 411: Clockwise wave
- 412: Counter-clockwise wave
- 501: Processing unit
- 502: Processing unit
- 503: Sender
- 504: Hollow conductor
- 505: Gantry
- 506: Processing unit
- 507: Processing unit
- 508: Receiver
- 509: Receiver
- 513: Electromagnetic shield

## Claims

1. A device for a computer tomography gantry (91) for transmitting data, wherein the gantry (91) comprises
- a stationary part (92),
- a rotary part (93),
wherein the device is adapted to transmit the data between the stationary part (92) of the gantry (91) and the rotary part (93) of the gantry (91),
wherein the device comprises
- a hollow conductor (104, 204, 308), which is adapted to guide a first wave,
- a sender (102, 103), which is adapted to send the first wave inside the hollow conductor, and
- a receiver (106), which is adapted to receive the first wave after a runtime inside the hollow conductor (104, 204, 308),
wherein the device is further adapted to transmit a second wave inside the hollow conductor (104, 204, 308), wherein the first wave runs clockwise and the second wave runs counter-clockwise, wherein the device is adapted to measure the runtimes of the first wave and the second wave

2. The device according to claim 1, wherein the hollow conductor (104, 204, 308) is arranged at one of the stationary part and the rotary part of the gantry (92), wherein the gantry comprises an inner bore, wherein the hollow conductor (104, 204, 308) is arranged around the inner bore of the gantry.

3. The device according to claim one of the preceding claims, wherein the device is adapted to calculate the distance between the receiver (106) and the sender (102, 103) by processing the runtimes of the first wave and the second wave.

4. The device according to claim 3, wherein the device is adapted to repeat the calculation of the distance, wherein the device is adapted to calculate the rotation speed of the rotary part of the gantry.

5. The device according to one of the preceding claims, wherein the first wave comprises data, wherein the device is adapted to receive the first wave after a runtime inside the hollow conductor (104, 204, 308), wherein the device is adapted to extract the data from the first wave after a runtime of the first wave in the hollow conductor (104, 204, 308).

6. The device according to one of the preceding claims, wherein the hollow conductor (104, 204, 308) is adapted to dampen the first wave, wherein the hollow conductor (104, 204, 308) is adapted to diminish an amplitude of the first wave after one circulation around the hollow conductor (104, 204, 308) considerably.

7. The device according to 6, wherein the hollow conductor (104, 204, 308) comprises a material which is adapted to diminish the amplitude of the first wave after one circulation around the hollow conductor (104, 204, 308) considerably.

8. The device according to one of the claims 6 or 7, wherein the hollow conductor (104, 204, 308) is at least partly coated in the interior zone.

9. The device according to claim 8, wherein at least a part of the coating of the hollow conductor (104, 204, 308) is of homogeneous chemical composition.

10. The device according to one of the claims 6 to 9, wherein the hollow conductor (104, 204, 308) is adapted to diminish the amplitude of the first wave with a first frequency after one circulation around the hollow conductor (104, 204, 308) considerably, wherein the hollow conductor (104, 204, 308) is adapted not to diminish the amplitude of a second wave with a second frequency after one circulation around the hollow conductor (104, 204, 308) considerably, wherein the first frequency is different to the second frequency.

11. The device according to one of the preceding claims, wherein the device is adapted to determine a relative position between the stationary part (92) and the rotary part (93) based on a measurement of the first wave running from the sender (102, 103) to the receiver (106).

12. The device according to one of the preceding claims, wherein the sender (403) and the receiver (403) are both provided at the same one of the stationary part and the rotary part (405) and wherein a reflector (410) is provided at the other of the stationary part and the rotary part (405) and wherein the device is adapted to determine a relative position between the stationary part and the rotary part based on a measurement of the first wave running from the sender (403) to the reflector (410) and back to the receiver (403).

13. The device according to one of the preceding claims, wherein the device comprises two receivers (509, 509') and an electromagnetic shield (513) positioned between the two receivers (508, 509), wherein a first receiver (508) is adapted to detect a first wave running from the sender (503) to the first receiver (508) in a clockwise direction and a second receiver (509) is adapted to detect a second wave running from the sender (503) to the second receiver (509) in a counter-clockwise direction.

14. A computer tomography gantry (91) comprising a device according to one of the preceding claims.

## Patentansprüche

1. Einrichtung für eine Computertomographie-Gantry (91) zur Datenübertragung,
wobei die Gantry (91) Folgendes umfasst:
- einen stationären Teil (92),
- einen rotierenden Teil (93),
wobei die Einrichtung so ausgelegt ist, dass sie die Daten zwischen dem stationären Teil (92) der Gantry (91) und dem rotierenden Teil (93) der Gantry (91) überträgt,
wobei die Einrichtung Folgendes umfasst:
- einen Hohlleiter (104, 204, 308), der so ausgelegt ist, dass er eine erste Welle leitet,
- einen Sender (102, 103), der so ausgelegt ist, dass er die erste Welle im Innern des Hohlleiters sendet, und
- einen Empfänger (106), der so ausgelegt ist, dass er die erste Welle nach einer Laufzeit im Innern des Hohlleiters (104, 204, 308) empfängt,
wobei die Einrichtung ferner so ausgelegt ist, dass sie eine zweite Welle im Innern des Hohlleiters (104, 204, 308) überträgt, wobei die erste Welle im Uhrzeigersinn und die zweite Welle entgegen dem Uhrzeigersinn läuft, wobei die Einrichtung so ausgelegt ist, dass sie die Laufzeiten der ersten und der zweiten Welle misst.

2. Einrichtung nach Anspruch 1, wobei der Hohlleiter (104, 204, 308) entweder an dem stationären oder an dem rotierenden Teil der Gantry (92) angeordnet ist, wobei die Gantry eine innere Öffnung umfasst, wobei der Hohlleiter (104, 204, 308) um die innere Öffnung der Gantry herum angeordnet ist.

3. Einrichtung nach einem der vorhergehenden Ansprüche, wobei die Einrichtung so ausgelegt ist, dass sie den Abstand zwischen dem Empfänger (106) und dem Sender (102, 103) durch Verarbeiten der Laufzeiten der ersten Welle und der zweiten Welle berechnet.

4. Einrichtung nach Anspruch 3, wobei die Einrichtung so ausgelegt ist, dass sie die Berechnung des Abstands wiederholt, wobei die Einrichtung so ausgelegt ist, dass sie die Drehgeschwindigkeit des rotierenden Teils der Gantry berechnet.

5. Einrichtung nach einem der vorhergehenden Ansprüche, wobei die erste Welle Daten umfasst, wobei die Einrichtung so ausgelegt ist, dass sie die erste Welle nach einer Laufzeit im Innern des Hohlleiters (104, 204, 308) empfängt, wobei die Einrichtung so ausgelegt ist, dass sie die Daten aus der ersten Welle nach einer Laufzeit der ersten Welle in dem Hohlleiter (104, 204, 308) extrahiert.

6. Einrichtung nach einem der vorhergehenden Ansprüche, wobei der Hohlleiter (104, 204, 308) so ausgelegt ist, dass er die erste Welle dämpft, wobei der Hohlleiter (104, 204, 308) so ausgelegt ist, dass er die Amplitude der ersten Welle nach einem Umlauf um den Hohlleiter (104, 204, 308) erheblich verringert.

7. Einrichtung nach Anspruch 6, wobei der Hohlleiter (104, 204, 308) ein Material umfasst, das so ausgelegt ist, dass es die Amplitude der ersten Welle nach einem Umlauf um den Hohlleiter (104, 204, 308) erheblich verringert.

8. Einrichtung nach einem der Ansprüche 6 oder 7, wobei der Hohlleiter (104, 204, 308) innen zumindest teilweise beschichtet ist.

9. Einrichtung nach Anspruch 8, wobei zumindest ein Teil der Beschichtung des Hohlleiters (104, 204, 308) eine homogene chemische Zusammensetzung aufweist.

10. Einrichtung nach einem der Ansprüche 6 bis 9, wobei der Hohlleiter (104, 204, 308) so ausgelegt ist, dass er die Amplitude der ersten Welle mit einer ersten Frequenz nach einem Umlauf um den Hohlleiter (104, 204, 308) erheblich verringert, wobei der Hohlleiter (104, 204, 308) so ausgelegt ist, dass er die Amplitude der zweiten Welle mit einer zweiten Frequenz nach einem Umlauf um den Hohlleiter (104, 204, 308) nicht erheblich verringert, wobei sich die erste Frequenz von der zweiten Frequenz unterscheidet.

11. Einrichtung nach einem der vorhergehenden Ansprüche, wobei die Einrichtung so ausgelegt ist, dass sie basierend auf einer Messung der vom Sender (102, 103) zum Empfänger (106) laufenden ersten Welle die relative Lage zwischen dem stationären Teil (92) und dem rotierenden Teil (93) ermittelt.

12. Einrichtung nach einem der vorhergehenden Ansprüche, wobei sich der Sender (403) und der Empfänger (403) beide auf dem gleichen entweder dem stationären Teil oder dem rotierenden Teil (405) befinden und wobei sich ein Reflektor (410) auf dem anderen entweder dem stationären Teil oder dem rotierenden Teil (405) befindet und wobei die Einrichtung so ausgelegt ist, dass sie die relative Lage zwischen dem stationären Teil und dem rotierenden Teil basierend auf einer Messung der vom Sender (403) zum Reflektor (410) und zurück zum Empfänger (403) laufenden ersten Welle ermittelt.

13. Einrichtung nach einem der vorhergehenden Ansprüche, wobei die Einrichtung zwei Empfänger (509, 509') und eine zwischen den beiden Empfängern (508, 509) angeordnete elektromagnetische Abschirmung (513) umfasst, wobei der erste Empfänger (508) so ausgelegt ist, dass er eine vom Sender (503) zum ersten Empfänger (508) im Uhrzeigersinn laufende erste Welle erkennt, und wobei der zweite Empfänger (509) so ausgelegt ist, dass er eine vom Sender (503) zum zweiten Empfänger (509) entgegen dem Uhrzeigersinn laufende zweite Welle erkennt.

14. Computertomographie-Gantry (91) mit einer Einrichtung nach einem der vorhergehenden Ansprüche.

## Revendications

1. Dispositif pour un portique de tomodensitométrie (91) pour transmettre des données, dans lequel le portique (91) comprend :
- une partie stationnaire (92),
- une partie rotative (93),
dans lequel le dispositif est adapté pour transmettre les données entre la partie stationnaire (92) du portique (91) et la partie rotative (93) du portique (91),
dans lequel le dispositif comprend
- un conducteur creux (104, 204, 308), qui est adapté pour guider une première onde,
- un émetteur (102, 103), qui est adapté pour envoyer la première onde à l'intérieur du conducteur creux, et
- un récepteur (106), qui est adapté pour recevoir la première onde après un temps de déplacement à l'intérieur du conducteur creux (104, 204, 308), dans lequel le dispositif est en outre adapté pour transmettre une seconde onde à l'intérieur du conducteur creux (104, 204, 308), dans lequel la première onde se déplace dans le sens des aiguilles d'une montre et la seconde onde se déplace dans le sens inverse des aiguilles d'une montre, dans lequel le dispositif est adapté pour mesurer les temps de déplacement de la première onde et de la seconde onde.

2. Dispositif selon la revendication 1, dans lequel le conducteur creux (104, 204, 308) est agencé sur une partie parmi la partie stationnaire et la partie rotative du portique (92), dans lequel le portique comprend un alésage intérieur, dans lequel le conducteur creux (104, 204, 308) est agencé autour de l'alésage intérieur du portique.

3. Dispositif selon la revendication une des revendications précédentes, dans lequel le dispositif est adapté pour calculer la distance entre le récepteur (106) et l'émetteur (102, 103) en traitant les temps de déplacement de la première onde et de la seconde onde.

4. Dispositif selon la revendication 3, dans lequel le dispositif est adapté pour répéter le calcul de la distance, dans lequel le dispositif est adapté pour calculer la vitesse de rotation de la partie rotative du portique.

5. Dispositif selon une des revendications précédentes, dans lequel la première onde comprend des données, dans lequel le dispositif est adapté pour recevoir la première onde après un temps de déplacement à l'intérieur du conducteur creux (104, 204, 308), dans lequel le dispositif est adapté pour extraire les données à partir de la première onde après un temps de déplacement de la première onde dans le conducteur creux (104, 204, 308).

6. Dispositif selon une des revendications précédentes, dans lequel le conducteur creux (104, 204, 308) est adapté pour amortir la première onde, dans lequel le conducteur creux (104, 204, 308) est adapté pour diminuer considérablement une amplitude de la première onde après une circulation autour du conducteur creux (104, 204, 308).

7. Dispositif selon la revendication 6, dans lequel le conducteur creux (104, 204, 308) comprend un matériau qui est adapté pour diminuer considérablement l'amplitude de la première onde après une circulation autour du conducteur creux (104, 204, 308).

8. Dispositif selon une des revendications 6 ou 7, dans lequel le conducteur creux (104, 204, 308) comprend un revêtement au moins partiel dans la zone intérieure.

9. Dispositif selon la revendication 8, dans lequel au moins une partie du revêtement du conducteur creux (104, 204, 308) est de composition chimique homogène.

10. Dispositif selon une des revendications 6 à 9, dans lequel le conducteur creux (104, 204, 308) est adapté pour diminuer considérablement l'amplitude de la première onde avec une première fréquence après une circulation autour du conducteur creux (104, 204, 308), dans lequel le conducteur creux (104, 204, 308) est adapté pour ne pas diminuer considérablement l'amplitude d'une seconde onde avec une seconde fréquence après une circulation autour du conducteur creux (104, 204, 308), dans lequel la première fréquence est différente de la seconde fréquence.

11. Dispositif selon une des revendications précédentes, dans lequel le dispositif est adapté pour déterminer une position relative entre la partie stationnaire (92) et la partie rotative (93) en fonction d'une mesure de la première onde se déplaçant de l'émetteur (102, 103) au récepteur (106).

12. Dispositif selon une des revendications précédentes, dans lequel l'émetteur (403) et le récepteur (403) sont tous les deux prévus dans la même partie parmi partie stationnaire et la partie rotative (405) et dans lequel un réflecteur (410) est prévu dans l'autre partie parmi la partie stationnaire et la partie rotative (405) et dans lequel le dispositif est adapté pour déterminer une position relative entre la partie stationnaire et la partie rotative en fonction d'une mesure de la première onde se déplaçant de l'émetteur (403) au réflecteur (410) et de retour au récepteur (403).

13. Dispositif selon une des revendications précédentes, dans lequel le dispositif comprend deux récepteurs (509, 509') et un blindage électromagnétique (513) positionné entre les deux récepteurs (508, 509), dans lequel un premier récepteur (508) est adapté pour détecter une première onde se déplaçant de l'émetteur (503) au premier récepteur (508) dans le sens des aiguilles d'une montre et un second récepteur (509) est adapté pour détecter une seconde onde se déplaçant de l'émetteur (503) au second récepteur (509) dans le sens inverse des aiguilles d'une montre.

14. Portique de tomodensitométrie (91) comprenant un dispositif selon une des revendications précédentes.
